(19) Europäisches Patentamt
European Patent Office
Office européen des brevets

(11) **EP 3 355 995 B1**

(12) **EUROPÄISCHE PATENTSCHRIFT**

(45) Veröffentlichungstag und Bekanntmachung des Hinweises auf die Patenterteilung:
**03.03.2021** Patentblatt **2021/09**

(21) Anmeldenummer: **16766565.2**

(22) Anmeldetag: **19.09.2016**

(51) Int Cl.:
*A61Q 19/00* (2006.01)    *A45D 34/00* (2006.01)
*A45D 40/00* (2006.01)    *A61K 8/06* (2006.01)
*A61K 8/81* (2006.01)

(86) Internationale Anmeldenummer:
**PCT/EP2016/072106**

(87) Internationale Veröffentlichungsnummer:
**WO 2017/055118 (06.04.2017 Gazette 2017/14)**

(54) **WEICH VERPACKTE KOSMETISCHE ZUBEREITUNG**

SOFT PACKED COSMETIC COMPOSITION

COMPOSITION COSMETIQUE DOUX EMBALLÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.10.2015 DE 102015219066**

(43) Veröffentlichungstag der Anmeldung:
**08.08.2018** Patentblatt **2018/32**

(73) Patentinhaber: **Beiersdorf AG**
**20253 Hamburg (DE)**

(72) Erfinder:
• **ALANYA-ROUSSEAU, Esma**
**22769 Hamburg (DE)**
• **ECKERT, Julia**
**22303 Hamburg (DE)**
• **WISCHHÖFER, Svea**
**21109 Hamburg (DE)**
• **SCHULZE, Sabrina**
**22081 Hamburg (DE)**
• **MEYER, Christiane**
**20357 Hamburg (DE)**
• **VON DAVIER, Anabelle**
**22391 Hamburg (DE)**
• **HETZEL, Frank**
**21261 Welle (DE)**

(56) Entgegenhaltungen:
EP-A1- 1 602 359    EP-A2- 1 513 491
EP-A2- 2 363 276    WO-A1-00/12309
WO-A1-2014/099305    CN-A- 1 753 776
US-A1- 2015 203 252

**Beschreibung**

Die vorliegende Erfindung betrifft ein Kosmetikum aus einer kosmetischen Zubereitung enthaltend ein oder mehrere bei 20 °C flüssiger Öle mit einem Spreitwert von weniger als 550 mm/10 min. sowie einem Packmittel aus HDPE, LDPE oder PP in Kombination mit 1-Propen-Ethylen-Copolymer,
dadurch gekennzeichnet, dass das Packmittel aus zwei Schichten aufgebaut ist, wobei die der Zubereitung zugewandte, innere Schicht aus HDPE, LDPE oder PP gebildet wird, während die äußere Schicht aus einer Mischung von HDPE, LDPE oder PP und in Kombination mit 1-Propen-Ethylen-Copolymer gebildet wird, mit der Maßgabe, dass in der äußeren Schicht immer das gleiche Polymer (HDPE, LDPE oder PP) verwendet, aus dem auch die innere Schicht besteht.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte werden in der Regel in Packmitteln in Form von Flaschen oder Tiegeln aufbewahrt. Diese bestehen überwiegend aus Glas (Glas-Tiegel) oder Kunststoffen, insbesondere aus reinem Polyethylen hoher Dichte (HDPE) oder reinem Polypropylen. Nachteilig an derartigen an sich sehr günstigen und umweltfreundlichen Packmitteln ist der Umstand, dass das Material relativ steif und unflexibel ist, so dass es regelmäßig Schwierigkeiten bereitet, insbesondere höher-viskose Zubereitungen zu entnehmen und die Packungen möglichst restlos zu entleeren.

Neben den Flaschen und Tiegeln kennt der Fachmann darüber hinaus die Packmittelform der Tube aus Metall oder Kunststoff, deren Material deutlich weicher und flexibler ist, als das bei Flaschen und Tiegeln der Fall ist. In der Kosmetik finden dabei vor allen, aufgrund ihres Gewichtes, ihres Preises, ihrer Korrosionsbeständigkeit und ihres Produkteindruckes auf den Verbraucher (derartige Packmittel mit "Soft-Touch-Haptik werden gerne mit Zubereitungen kombiniert, die ein weiches Hautgefühl vermitteln, damit die welche Oberfläche des Packmittels den Produkteindruck im Sinne eines holistischen Produktkonzeptes unterstreicht) die Kunststoff-Tuben Verwendung. Diese haben jedoch den Nachteil, dass sie, wenn die in Ihr abgefüllte kosmetische Zubereitung ein oder mehrere Ölkomponenten enthält, nicht besonders lagerbeständig sind sondern vielmehr bei Kontakt mit dem Öl aufquellen, da das Öl vom Kunststoff "aufgesaugt" und absorbiert wird. Darüber hinaus können die Produkte Ausölan. Nicht zuletzt führt das Öl im Kunststoff dazu, dass auf dem Packmittel aufgedruckte Beschriftungen verwischen und sich ablösen können. Aus diesen Gründen kann in derartigen Packmitteln entweder nur ölfreie Zubereitungen (z.B. Zahnpasta) abgefüllt werden oder das Packmittel muss auf der der kosmetischen Zubereitung zugewandten Innenseite aufwändig beschichtet bzw. versiegelt werden. Aus dem Stand der Technik bekannt sind beispielsweise Schutzbarrieren aus Ethylenvinylalkohol, die relativ teuer und weniger gut zu recyceln sind als reine Polyolefin-Packmittel.

Es war daher die Aufgabe der vorliegenden Erfindung, ein Kosmetikum aus einer ölhaltigen kosmetischen Zubereitung und einer welch-flexiblen ("soft-Touch" Haptik) Kunststoffverpackung zu entwickeln, die lagerstabil ist, d.h. bei dem die Ölabsorption der Kunststoffverpackung deutlich reduziert ist, ohne das auf dem Kunststoff eine Schutzschicht (z.B. aus Ethylenvinylalkohol) aufgetragen werden muss.

Überraschend gelöst wird die Aufgabe durch ein Kosmetikum aus

a) einer kosmetischen Zubereltung enthaltend ein oder mehrere bei 20 °C flüssiger Öle mit einem Spreitwert von weniger als 550 mm/10 min. sowie

b) einem Packmittel aus HDPE (= Polyethylen hoher Dichte), LOPE (= Polyethylen niedriger Dichte) oder PP (=Polypropylen) in Kombination mit 1-Propen-Ethylen-Copolymer.

Alle Aggregatszustände und Temperaturangaben beziehen sich auf Bestimmungen bei Normaldruck (1,013 bar).

Der erfindungsgemäße Spreitwert wird dabei wie folgt bestimmt:

Messtemperatur: 20 °C,

Geräte/Hilfsmittel: Eppendorf-Pipette (Multipette VWR-Nr.: 613-3669), Eppendorf-Kombitips grün (VWR-Nr.: 613-2062), rundes Filterpapier Whatman 589/2 110mm ashless/white ribbon (VWR-Nr.: 512-3608).

Durchführung:

Ein Filterpapier wird auf den Labor - Arbeitstisch gelegt (ohne weitere Unterlage), die Pipette auf 25 μl eingestellt und das zu vermessende Lipid aufgezogen. Anschließend wird die Pipette von außen mit einem Papiertuch abgewischt. 25 μl des zu vermessenden Lipides werden zentral aus einer Höhe von ca. 30 mm auf das Filterpapier appliziert und die Einwirkzeit mit einer Stoppuhr gemessen. Nach 10 Minuten Spreitzeit wird der entstandene Fleck sofort mit einem Bleistift umrandet. Diese Messung wird auf fünf Filterpapieren für ein Lipid durchgeführt. Vom umrandeten Fleck wird an drei verschiedenen Stellen der Durchmesser ermittelt in mm. Um den Durchschnittswert zu bekommen, werden die 15 Werte (5 Filterpapiere mit jeweils 3 Durchmessern) addiert und dann durch 15 geteilt. Mit diesem Durchschnittswert wird dann die Fläche des gespreiteten Lipids berechnet (Flächenberechnung eines Kreises):

$$A = \pi * r^2 \quad \text{oder } A = \pi * \left(\frac{d}{2}\right)^2$$

Der Spreitwert wird für 25 μL in mm$^2$/10 min. angegeben.

Die folgende Tabelle gibt einige Spreitwerte an, die nach dieser Methode bestimmt wurden:

| INCI | Spreitwert (mm$^2$/10 min) |
|---|---|
| Olus Oil | 434 |
| Cocoglycerides | 460 |
| Octyldodecanol | 531 |
| Caprylic/Capric Triglyceride | 515 |
| Dicaprylyl Ether | 995 |
| C15-19 Alkane | 951 |
| Isopropyl Palmitate | 855 |
| Octocrylene | 113 |
| Decyl Oleate | 726 |
| Ethylhexyl Stearate | 647 |
| Isopropyl Stearate | 824 |
| Dimethicone | 353 |
| C12-15 Alkyl Benzoate | 750 |

Es ist erfindungsgemäß vorteilhaft, wenn die bei 20 °C flüssigen Öle mit einem Spreitwert von weniger als 550 mm/10 min. gewählt werden aus der Gruppe der Verbindungen mit der INCI Olus Oil, Cocoglycerides, Octyldodecanol, Caprylic/Capric Triglyceride, Octocrylene, Dimethicone, Ethanol.

Darüber hinaus sind erfindungsgemäß vorteilhafte Ausführungsformen der vorliegenden Erfindung dadurch gekennzeichnet, dass die Zubereitung frei ist von bei 20 °C flüssigen Ölen gewählt aus der Gruppe der Verbindungen mit der INCI Dicaprylyl Ether, C15-19 Alkane, Isopropyl Palmitate, Decyl Oleate, Tridecyl Sterarate, Tridecyl Trimelliate, Dipentaerythityl Hexacaprylate/Hexacaprate, Ethylhexyl Stearate, Isopropyl Stearate, C12-15 Alkyl Benzoate.

Es ist vorteilhaft im Sinne der vorliegenden Erfindung, wenn die kosmetische Zubereitung die bei 20 °C flüssigen Öle mit einem Spreitwert von weniger als 550 mm/10 min. in einer Menge von mindestens 1 Gewichts-%, bezogen auf das Gesamtgewicht der Zubereitung, enthält.

Es ist bevorzugt im Sinne der vorliegenden Erfindung, wenn die kosmetische Zubereitung die bei 20 °C flüssigen Öle mit einem Spreitwert von weniger als 550 mm/10 min. in einer Menge von 0,1 bis 10 Gewichts-%, bezogen auf das Gesamtgewicht der Zubereitung, enthält.

Es ist erfindungsgemäß vorteilhaft, wenn Olus Oil in einer Menge von 0,1 bis 10 Gewichts-%, bezogen auf das Gesamtgewicht der Zubereitung, in dieser eingesetzt wird.

Es ist erfindungsgemäß vorteilhaft, wenn Cocoglyceride in einer Menge von 0,1 bis 10 Gewichts-%, bezogen auf das Gesamtgewicht der Zubereitung, in dieser eingesetzt werden.

Es ist erfindungsgemäß vorteilhaft, wenn Octyldodecanol in einer Menge von 0,1 bis 10 Gewichts-%, bezogen auf das Gesamtgewicht der Zubereitung, in dieser eingesetzt wird.

Es ist erfindungsgemäß vorteilhaft, wenn Caprylic/Capric Triglyceride in einer Menge von 0,1 bis 10 Gewichts-%, bezogen auf das Gesamtgewicht der Zubereitung, in dieser eingesetzt wird.

Es ist erfindungsgemäß vorteilhaft, wenn Octocrylen in einer Menge von 0,1 bis 10 Gewichts-%, bezogen auf das Gesamtgewicht der Zubereitung, in dieser eingesetzt wird.

Es ist erfindungsgemäß vorteilhaft, wenn Dimethicone in einer Menge von 0,1 bis 10 Gewichts-%, bezogen auf das Gesamtgewicht der Zubereitung, in dieser eingesetzt wird.

Es ist erfindungsgemäß vorteilhaft, wenn Ethanol in einer Menge von 0,1 bis 10 Gewichts-%, bezogen auf das Gesamtgewicht der Zubereitung, in dieser eingesetzt wird.

Erfindungsgemäß besonders bevorzugt ist es, wenn die erfindungsgemäße Zubereitung eine Kombination aus Ethanol und einem oder mehreren Ölen aus der Gruppe der Verbindungen Olus Oil, Cocoglycerides, Octyldodecanol, Caprylic/Capric Triglyceride, Octocrylene, Dimethicone enthält.

Erfindungsgemäß vorteilhafte Ausführungsformen der vorliegenden Erfindung sind auch dadurch gekennzeichnet, dass die kosmetische Zubereitung Glycerin enthält.

In einem solchen Falle ist es erfindungsgemäß von Vorteil, wenn der Gehalt an Glycerin von 0,1 bis 30 Gewichts-%, bezogen auf das Gesamtgewicht der Zubereitung, beträgt.

Erfindungsgemäß vorteilhaft ist es ferner, wenn die kosmetische Zubereitung in Form einer Emulsion vorliegt. Dabei ist es erfindungsgemäß bevorzugt, wenn die kosmetische Zubereitung in Form einer O/W-Emulsion vorliegt.

Darüber hinaus sind diese Emulsionen als erfindungsgemäß vorteilhaft dadurch gekennzeichnet, dass die Zubereitung einen oder mehrere Emulgatoren gewählt aus der Gruppe der Verbindungen Glycerylstearatcitrat, Cetearylalkohol, Natriumcetearylsulfat + Glycerylstearat, Cetearylsulfosuccinat, Natriumstearoylglutamat, Polyglyceryl-3-methylglucosedistearat, Stearinsäure, Polyglyceryl-10 Stearat, Polyglyceryl-4 Diisostearate/Polyhydroxystearate/Sebacate Kaliumcetylphosphat, enthält.

Die Emulgatorkonzentration (Gesamtkonzentration) beträgt dabei vorteilhaft von 0,1 bis 3% Gewichts-%, bezogen auf das Gesamtgewicht der Zubereitung.

Darüber hinaus kann die erfindungsgemäße Zubereitung die für solche Zusammensetzungen üblichen Inhaltsstoffe enthalten.

Enthält die erfindungsgemäße Zusammensetzung einen oder mehrere Füllstoffe wie beispielsweise Silikate, Calcit oder Magnesiumcarbonat, so sind diese Füllstoffe höchstens in einer Menge von 5 Gewichts-%, bezogen auf das Gesamtgewicht der Zubereitung, enthalten.

Ferner ist es erfindungsgemäß vorteilhaft, wenn die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, Vitamin F, β-Alanin, Tocopherylacetat, Panthenol, Mandelöl, Shea Butter, Kakaobutter, Aloe Vera, Magnolol, Honokiol, Harnstoff, Hyaluronsäure, Dihydroxyaceton, 8-Hexadecen-1,16-dicarbonsäure, Glycyrrhetinsäure, Glucosylglyceride und/oder Licochalcon A enthält.

Außerdem sind erfindungsgemäß vorteilhafte Ausführungsformen der vorliegenden Erfindung dadurch gekennzeichnet, dass die Zubereitung Propylenglycol, Butylenglycol, 2-Methylpropan-1,3-diol, Ethylhexylglycerin, 1,2-Pentandiol, 1,2-Hexandiol, 1,2-Octandiol und/oder 1,2-Decandiol enthält. Dabei ist der Gehalt an 2-Methylpropan-1,3-diol, 1,2-Pentandiol und/oder 1,2-Hexandiol erfindungsgemäß bevorzugt.

Es ist erfindungsgemäß vorteilhaft, wenn die Zubereitung frei ist von Parabenen, Isothiazolinonen und 3-Iodpropargyl-*N*-butylcarbamat (IPBC).

Auch ist es erfindungsgemäß besonders vorteilhaft, wenn die erfindungsgemäße Zubereitung frei ist von Natriumlaurylethersulfat und Cocamidopropylbetainen.

Hingegen sind erfindungsgemäß vorteilhafte Ausführungsformen dadurch gekennzeichnet, dass die Emulsion Pirocton-Olamin (1-Hydroxy-4-methyl-6-(2,4,4-trimethylpentyl)- 2(1*H*)-pyridon monoethanolaminsalz) und/oder Phenoxyethanol enthält.

Enthält die Emulsion Phenoxyethanol, so ist es erfindungsgemäß von Vorteil, wenn der Gehalt an Phenoxyethanol von 0,1 bis 1 Gew.-%, bezogen auf das Gesamtgewicht der Emulsion beträgt.

Es ist erfindungsgemäß vorteilhaft, wenn die kosmetische Zubereitung eine Viskosität von größer/gleich 2000 mPas/s, aufweist. Dabei wird die erfindungsgemäße Viskosität wie folgt bestimmt: Messung bei 25°C im 150 ml Rollrandglas mittels Rheomat R123 von der Firma proRheo. Der Rheomat 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen das verwendete Messsystem wird die Viskosität berechnet. Messkörper Nr.3 (Artikelnr. 200 0193), geeignet für einen Viskositätsbereich bis 1.000 [mPas], Drehzahl Bereich 62,5 min

Erfindungsgemäß vorteilhaft beträgt pH-Wert der erfindungsgemäßen Zubereitung 4-9.

Das erfindungsgemäße Packmittel ist aus zwei Schichten aufgebaut, wobei die der Zubereitung zugewandte, innere Schicht aus HDPE, LDPE oder PP gebildet wird, während die äußere Schicht aus einer Mischung von HDPE, LDPE oder PP und in Kombination mit 1-Propen-Ethylen-Copolymer gebildet wird. Dabei wird in der äußeren Schicht immer das gleiche Polymer (HDPE, LDPE oder PP) verwendet, aus dem auch die innere Schicht besteht.

In einem solchen Falle ist es erfindungsgemäß bevorzugt, wenn die Kombination aus HDPE, LDPE oder PP mit 1-Propen-Ethylen-Copolymer in der äußeren Schicht ein Mischungsverhältnis (Gewichtsverhältnis) aufweist, bei dem der Gehalt an 1-Propen-Ethylen-Copolymer mindestens 50% beträgt. Bevorzugt ist beispielsweise das Mischungsverhältnis HDPE, LDPE oder PP zu 1-Propen-Ethylen-Copolymer von 40:60.

Es ist erfindungsgemäß besonders bevorzugt, wenn die Kombination aus HDPE, LDPE oder PP mit 1-Propen-Ethylen-Copolymer in der äußeren Schicht eine Mischung mit einem Gewichtsverhältnis von 50:50 darstellt.

Erfindungsgemäß besonders bevorzugt ist es in einem solchen Falle, wenn die beiden Schichten in einem Gewichtsverhältnis von 80 Gew.-% innere Schicht und 20 Gew.-% äußere Schicht gebildet wird.

Als HDPE (high density polyethylene) können alle handelsüblichen Polyethylene dieser Gruppe eingesetzt werden.

Als LDPE (low density polyethylene) können alle handelsüblichen Polyethylene dieser Gruppe eingesetzt werden.

Als PP (polypropylene) können alle handelsüblichen Polypropylene dieser Gruppe eingesetzt werden.

Bei dem 1-Propen-Ethylen-Copolymer handelt es sich erfindungsgemäß vorteilhaft um ein thermoplastisches Olefin.

Es ist dabei erfindungsgemäß von Vorteil, wenn dieses Copolymer eine Dichte (gemessen nach ISO 1183) von 0,89 g/cm$^3$ (+/- 10 %) aufweist.

Es ist darüber hinaus erfindungsgemäß von Vorteil, wenn dieses Copolymer eine Schmelzflussrate (Melt flow rate) bei 230 °C/2.16 Kg (gemessen nach ISO 1133) von 0,80 g/cm$^3$ (+/- 10 %) aufweist.

Es ist ferner erfindungsgemäß von Vorteil, wenn dieses Copolymer eine Streckspannung (Tensile Stress at Yield) (gemessen nach ISO 527-1,-2) von 9 MPa (+/- 10 %) aufweist.

Es ist nicht zuletzt erfindungsgemäß von Vorteil, wenn dieses Copolymer eine Reißdehnung (Tensile Strain at Break) (gemessen nach ISO 527-1,-2) von 500% (+/- 10 %) aufweist.

**Versuche**

Mit den folgenden Versuchen konnte die Stabilität des Packmittels gegenüber den Ölen nachgewiesen werden:

| | A | B |
|---|---|---|
| Zusammensetzung in % | m [%] | m [%] |
| Glyceryl Stearat SE | 1,5 | 1,5 |
| Stearinsäure* | 2,5 | 2,5 |
| Cetyl Palmitat | 2,5 | 2,5 |
| Shea Butter | 1,0 | 1,0 |
| Myristyl Myristat | 2 | 2 |
| Octyldodecanol | | 10,00 |
| Dicaprylyl Ether | 10,00 | |
| Dimethicon | 1,0 | 1,0 |
| Carbomer | 0,2 | 0,2 |
| Glycerin | 5,0 | 5,0 |
| Natronlauge q.s. auf pH Wert | pH 6 | pH 6 |
| Phenoxyethanol | 0,90 | 0,90 |
| Cetearyl Alcohol | 3,0 | 3,0 |
| Wasser | ad 100 | ad 100 |
| Formelstabilität nach 1 monatiger Lagerung bei 40°C, Packmittel: LDPE in Kombination mit 1-Propen-Ethylen-Copolymer** | Quellung/Deformieru ng des Packmittels | stabil |

(fortgesetzt)

|  | A | B |
|---|---|---|
| Zusammensetzung in % | m [%] | m [%] |
| Formelstabilität nach 2 monatiger Lagerung bei 40°C, Packmittel: LDPE in Kombination mit 1-Propen-Ethylen-Copolymer** | Deutliche Quellung/ Deformieru ng des Packmittels | stabil |

* Mischung Palmitic Acid + Stearic Acid + Myristic Acid + Arachidic Acid + Oleic Acid **=2 Schichtenaufbau, innere Schicht: LDPE, äußere Schicht LDPE+1-Propen-Ethylen-Copolymer im Gew.-Verhältnis 50:50: Gewichtsverhältnis Schichten: 80% innere Schicht, 20% äußere Schicht. 1-Propen-Ethylen-Copolymer: Dichte: 0,89 g/cm$^3$, Schmelzflussrate: 0,80 g/cm$^3$, Streckspannung: 9 MPa, Reißdehnung: 500%.

**Beispiele**

Die nachfolgenden Beispiele sollen die vorliegende Erfindung verdeutlichen, ohne sie einzuschränken. Alle Mengenangaben, Anteile und Prozentanteile sind auf das Gewicht und die Gesamtmenge bzw. auf das Gesamtgewicht der Zubereitungen bezogen.

| | C | D | E | F |
|---|---|---|---|---|
| Zusammensetzung in % | m [%] | m [%] | m [%] | m [%] |
| Glyceryl Stearat SE | 1,5 | 1,5 | 1,5 | 1,5 |
| Stearinsäure* | 2,5 | 2,0 | 1,0 | 1,0 |
| Cetyl Palmitat | 2,5 | 2,5 | 2,0 | 2,0 |
| Shea Butter | 1,0 | 2,0 | 2,0 | 2,0 |
| Myristyl Myristat | 2 | 2 | | |
| Octyldodecanol | 10,00 | 6,00 | 2,00 | |
| Dimethicone | 1,0 | 0,5 | 2,0 | 2,0 |
| Caprylic/Capric Triglycerid | | | 4,0 | 8,0 |
| Carbomer | 0,2 | 0,2 | 0,2 | 0,2 |
| Glycerin | 5,0 | 8,0 | 8,0 | 8,0 |
| Phenoxyethanol | 0,90 | 0,70 | 0,70 | 0,70 |
| Cetearyl Alcohol | 3,0 | 3,0 | 2,0 | 2,0 |
| Ethylhexylglycerin | | 0,1 | 0,1 | 0,1 |
| Natrium EDTA | | 0,25 | 0,25 | 0,25 |
| Coenzym Q10 | | | 1 | 1 |
| Etahnol | | | 3 | |
| Octocrylen | | | 1 | 1 |
| Butyl Methoxydibenzoylmethan | | | 1 | 1 |
| Tapiokastärke | | | | 1 |
| Natronlauge q.s. auf pH Wert | pH 5,5 | pH 6,0 | pH 7,0 | pH 7,1 |
| Wasser | ad 100 | ad 100 | ad 100 | ad 100 |

**EP 3 355 995 B1**

(fortgesetzt)

| Zusammensetzung in % | C | D | E | F |
|---|---|---|---|---|
|  | m [%] | m [%] | m [%] | m [%] |
| Packmittel | LDPE in Kombination mit 1-Propen-Ethylen-Copolymer** | HDPE in Kombination mit 1-Propen-Ethylen-Copolymer** | LDPE in Kombination mit 1-Propen-Ethylen-Copolymer** | PP in Kombination mit 1-Propen-Ethylen-Copolymer** |

\* Mischung Palmitic Acid + Stearic Acid + Myristic Acid + Arachidic Acid + Oleic Acid
\*\*=2 Schichtenaufbau, innere Schicht: Polypolefin, äußere Schicht Polyolefin +1-Propen-Ethylen-Copolymer im Gew.-Verhältnis 50:50: Gewichtsverhältnis Schichten: 80% innere Schicht, 20% äußere Schicht. 1-Propen-Ethylen-Copolymer: Dichte: 0,89 $g/cm^3$, Schmelzflussrate: 0,80 $g/cm^3$, Streckspannung: 9 MPa, Reißdehnung: 500%.

**Patentansprüche**

1. Kosmetikum aus

   a) einer kosmetischen Zubereitung enthaltend ein oder mehrere bei 20 °C flüssiger Öle mit einem Spreitwert von weniger als 550 mm/10 min. sowie
   b) einem Packmittel aus HDPE, LDPE oder PP in Kombination mit 1-Propen-Ethylen-Copolymer, **dadurch gekennzeichnet, dass** das Packmittel aus zwei Schichten aufgebaut ist, wobei die der Zubereitung zugewandte, innere Schicht aus HDPE, LDPE oder PP gebildet wird, während die äußere Schicht aus einer Mischung von HDPE, LDPE oder PP und in Kombination mit 1-Propen-Ethylen-Copolymer gebildet wird, mit der Maßgabe, dass in der äußeren Schicht immer das gleiche Polymer (HDPE, LDPE oder PP) verwendet, aus dem auch die innere Schicht besteht.

2. Kosmetikum nach Anspruch 1, **dadurch gekennzeichnet, dass** die bei 20 °C flüssigen Öle mit einem Spreitwert von weniger als 550 mm/10 min. gewählt werden aus der Gruppe der Verbindungen mit der INCI Olus Oil, Cocoglycerides, Octyldodecanol, Caprylic/Capric Triglyceride, Octocrylene, Dimethicone.

3. Kosmetikum nach Anspruch 1, **dadurch gekennzeichnet, dass** die Zubereitung Ethanol enthält.

4. Kosmetikum nach einem der vorhergehenden Ansprüche, **dadurch gekennzeichnet, dass** die Zubereitung frei ist von bei 20 °C flüssigen Ölen gewählt aus der Gruppe der Verbindungen mit der INCI Dicaprylyl Ether, C15-19 Alkane, Isopropyl Palmitate, Decyl Oleate, Tridecyl Sterarate, Tridecyl Trimelliate, Dipentaerythityl Hexacaprylate/Hexacaprate, Ethylhexyl Stearate, Isopropyl Stearate, C12-15 Alkyl Benzoate.

5. Kosmetikum nach einem der vorhergehenden Ansprüche, **dadurch gekennzeichnet, dass** die kosmetische Zubereitung die bei 20 °C flüssigen Öle mit einem Spreitwert von weniger als 550 mm/10 min. in einer Menge von mindestens 1 Gewichts-%, bezogen auf das Gesamtgewicht der Zubereitung, enthält.

6. Kosmetikum nach einem der vorhergehenden Ansprüche, **dadurch gekennzeichnet, dass** die kosmetische Zubereitung die bei 20 °C flüssigen Öle mit einem Spreitwert von weniger als 550 mm/10 min. in einer Menge von 0,1 bis 10 Gewichts-%, bezogen auf das Gesamtgewicht der Zubereitung, enthält.

7. Kosmetikum nach einem der vorhergehenden Ansprüche, **dadurch gekennzeichnet, dass** die kosmetische Zubereitung Glycerin enthält.

8. Kosmetikum nach einem der vorhergehenden Ansprüche, **dadurch gekennzeichnet, dass** die kosmetische Zubereitung in Form einer Emulsion vorliegt.

9. Kosmetikum nach einem der vorhergehenden Ansprüche, **dadurch gekennzeichnet, dass** die kosmetische Zubereitung in Form einer O/W-Emulsion vorliegt.

10. Kosmetikum nach einem der vorhergehenden Ansprüche, **dadurch gekennzeichnet, dass** die kosmetische Zubereitung eine Viskosität von größer/gleich 2000 mPas/s aufweist, gemessen bei 25°C im 150 ml Rollrandglas mittels Rheomat R123 von der Firma proRheo mit Messkörper 3.

**11.** Kosmetikum nach einem der vorhergehenden Ansprüche, **dadurch gekennzeichnet, dass** die Kombination aus HDPE, LDPE oder PP mit 1-Propen-Ethylen-Copolymer eine Mischung mit einem Gewichtsverhältnis von 50:50 darstellt.

**12.** Kosmetikum nach einem der Ansprüche 10 oder 11, **dadurch gekennzeichnet, dass** die beiden Schichten in einem Gewichtsverhältnis von 80% innere Schicht und 20% äußere Schicht gebildet wird.

**13.** Kosmetikum nach einem der vorhergehenden Ansprüche, **dadurch gekennzeichnet, dass** das 1-Propen-Ethylen-Copolymer eine Dichte (gemessen nach ISO 1183) von 0,89 g/cm$^3$ (+/- 10 %) aufweist.

**14.** Kosmetikum nach einem der vorhergehenden Ansprüche, **dadurch gekennzeichnet, dass** das 1-Propen-Ethylen-Copolymer eine Schmelzflussrate (Melt flow rate) bei 230 °C/2.16 Kg (gemessen nach ISO 1133) von 0,80 g/cm$^3$ (+/- 10 %) aufweist.

**15.** Kosmetikum nach einem der vorhergehenden Ansprüche, **dadurch gekennzeichnet, dass** das 1-Propen-Ethylen-Copolymer eine Streckspannung (Tensile Stress at Yield) (gemessen nach ISO 527-1,-2) von 9 MPa (+/- 10 %) aufweist.

## Claims

**1.** Cosmetic comprising

a) a cosmetic preparation containing one or more oils that are liquid at 20°C and have a spreading value of less than 550 mm/10 min, and also

b) a packaging made of HDPE, LDPE or PP in combination with 1-propene-ethylene copolymer, **characterized in that** the packaging is assembled from two layers, wherein the inner layer which faces the preparation is formed of HDPE, LDPE or PP, while the outer layer is formed of a mixture of HDPE, LDPE or PP and in combination with 1-propene-ethylene copolymer, with the proviso that the same polymer (HDPE, LDPE or PP) of which the inner layer consists is always also used in the outer layer.

**2.** Cosmetic according to Claim 1, **characterized in that** the oils that are liquid at 20°C and have a spreading value of less than 550 mm/10 min are selected from the group of compounds with the INCI names Olus Oil, Cocoglycerides, Octyldodecanol, Caprylic/Capric Triglyceride, Octocrylene, Dimethicone.

**3.** Cosmetic according to Claim 1, **characterized in that** the preparation comprises ethanol.

**4.** Cosmetic according to any of the preceding claims, **characterized in that** the preparation is free from oils that are liquid at 20°C selected from the group of compounds with the INCI names Dicaprylyl Ether, C15-19 Alkane, Isopropyl Palmitate, Decyl Oleate, Tridecyl Stearate, Tridecyl Trimellitate, Dipentaerythrityl Hexacaprylate/Hexacaprate, Ethylhexyl Stearate, Isopropyl Stearate, C12-15 Alkyl Benzoate.

**5.** Cosmetic according to any of the preceding claims, **characterized in that** the cosmetic preparation contains the oils that are liquid at 20°C and have a spreading value of less than 550 mm/10 min in an amount of at least 1 wt%, based on the total weight of the preparation.

**6.** Cosmetic according to any of the preceding claims, **characterized in that** the cosmetic preparation contains the oils that are liquid at 20°C and have a spreading value of less than 550 mm/10 min in an amount from 0.1 to 10 wt%, based on the total weight of the preparation.

**7.** Cosmetic according to any of the preceding claims, **characterized in that** the cosmetic preparation contains glycerol.

**8.** Cosmetic according to any of the preceding claims, **characterized in that** the cosmetic preparation is in the form of an emulsion.

9. Cosmetic according to any of the preceding claims, **characterized in that** the cosmetic preparation is in the form of an O/W emulsion.

10. Cosmetic according to any of the preceding claims, **characterized in that** the cosmetic preparation has a viscosity of greater than/equal to 2000 mPas/s, measured at 25°C in a 150 ml rolled rim bottle using a Rheomat R123 from proRheo with measurement body no. 3.

11. Cosmetic according to any of the preceding claims, **characterized in that** the combination of HDPE, LDPE or PP with 1-propene-ethylene copolymer is a mixture with a weight ratio of 50:50.

12. Cosmetic according to either of Claims 10 and 11, **characterized in that** the two layers are formed in a weight ratio of 80% inner layer and 20% outer layer.

13. Cosmetic according to any of the preceding claims, **characterized in that** the 1-propene-ethylene copolymer has a density (measured according to ISO 1183) of 0.89 g/cm$^3$ (+/- 10%).

14. Cosmetic according to any of the preceding claims, **characterized in that** the 1-propene-ethylene copolymer has a melt flow rate at 230°C/2.16 kg (measured according to ISO 1133) of 0.80 g/cm$^3$ (+/- 10%).

15. Cosmetic according to any of the preceding claims, **characterized in that** the 1-propene-ethylene copolymer has a tensile stress at yield (measured according to ISO 527-1, 527-2) of 9 MPa (+/- 10%).

**Revendications**

1. Produit cosmétique composé

    a) d'une préparation cosmétique contenant une ou plusieurs huiles liquides à 20 °C comportant une valeur de dispersion inférieure à 550 mm/10 min, ainsi
    b) qu'un emballage d'HDPE, de LDPE ou de PP en combinaison avec un copolymère de 1-propène-éthylène, **caractérisé en ce que** l'emballage est construit de deux couches, la couche interne orientée vers la préparation étant formée d'HDPE, de LDPE ou de PP, tandis que la couche externe est formée d'un mélange d'HDPE, de LDPE ou de PP et en combinaison avec un copolymère de 1-propène-éthylène, à la condition que le même polymère (HDPE, LDPE ou PP) que celui de la couche interne soit toujours utilisé dans la couche externe.

2. Produit cosmétique selon la revendication 1, **caractérisé en ce que** les huiles liquides à 20 °C comportant une valeur de dispersion inférieure à 550 mm/10 min sont choisies dans le groupe des composés d'INCI Olus Oil, Cocoglycerides, Octyldodecanol, Caprylic/Capric Triglycéride, Octocrylene, Dimethicone.

3. Produit cosmétique selon la revendication 1, **caractérisé en ce que** la préparation contient de l'éthanol.

4. Produit cosmétique selon l'une quelconque des revendications précédentes, **caractérisé en ce que** la préparation est exempte d'huiles liquides à 20 °C choisies dans le groupe des composés d'INCI Dicaprylyl Ether, C15-19 Alkane, Isopropyl Palmitate, Decyl Oleate, Tridecyl Sterarate, Tridecyl Trimelliate, Dipentaerythityl Hexacaprylate/Hexacaprate, Ethylhexyl Stearate, Isopropyl Stearate, C12-15 Alkyl Benzoate.

5. Produit cosmétique selon l'une quelconque des revendications précédentes, **caractérisé en ce que** la préparation cosmétique contient les huiles liquides à 20 °C comportant une valeur de dispersion inférieure à 550 mm/10 min en une quantité d'au moins 1 % en poids, par rapport au poids total de la préparation.

6. Produit cosmétique selon l'une quelconque des revendications précédentes, **caractérisé en ce que** la préparation cosmétique contient les huiles liquides à 20 °C comportant une valeur de dispersion inférieure à 550 mm/10 min en une quantité de 0,1 à 10 % en poids, par rapport au poids total de la préparation.

7. Produit cosmétique selon l'une quelconque des revendications précédentes, **caractérisé en ce que** la préparation cosmétique contient de la glycérine.

8. Produit cosmétique selon l'une quelconque des revendications précédentes, **caractérisé en ce que** la préparation cosmétique est présente sous forme d'une émulsion.

9. Produit cosmétique selon l'une quelconque des revendications précédentes, **caractérisé en ce que** la préparation cosmétique est présente sous forme d'une émulsion huile/eau.

10. Produit cosmétique selon l'une quelconque des revendications précédentes, **caractérisé en ce que** la préparation cosmétique présente une viscosité supérieure ou égale à 2 000 mPas/s, mesurée à 25 °C dans un verre à bord roulé de 150 ml au moyen d'un Rheomat R123 de la société proRheo avec un corps de mesures 3.

11. Produit cosmétique selon l'une quelconque des revendications précédentes, **caractérisé en ce que** la combinaison d'HDPE, de LDPE ou de PP avec un copolymère de 1-propène-éthylène représente un mélange doté d'un rapport en poids de 50 : 50.

12. Produit cosmétique selon l'une quelconque des revendications 10 et 11, **caractérisé en ce que** les deux couches sont formées en un rapport en poids de 80 % de couche interne et 20 % de couche externe.

13. Produit cosmétique selon l'une quelconque des revendications précédentes, **caractérisé en ce que** le copolymère de 1-propène-éthylène présente une densité (mesurée selon la norme ISO 1183) de 0,89 g/cm$^3$ ($\pm$ 10 %).

14. Produit cosmétique selon l'une quelconque des revendications précédentes, **caractérisé en ce que** le copolymère de 1-propène-éthylène présente un indice de fluidité à chaud à 230 °C/2,16 kg (mesuré selon la norme ISO 1133) de 0,80 g/cm$^3$ ($\pm$ 10 %).

15. Produit cosmétique selon l'une quelconque des revendications précédentes, **caractérisé en ce que** le copolymère de 1-propène-éthylène présente une contrainte en traction (mesurée selon la norme ISO 527-1,-2) de 9 MPa ($\pm$ 10 %).